(19) Europäisches Patentamt
European Patent Office
Office européen des brevets

(11) Veröffentlichungsnummer: **0 090 240**
**A2**

# (12) EUROPÄISCHE PATENTANMELDUNG

(21) Anmeldenummer: **83102479.9**

(22) Anmeldetag: **14.03.83**

(51) Int. Cl.³: **C 07 D 501/59**, A 61 K 31/545 // C07D233/76

(30) Priorität: **25.03.82 DE 3211034**

(43) Veröffentlichungstag der Anmeldung: **05.10.83 Patentblatt 83/40**

(84) Benannte Vertragsstaaten: **AT BE CH DE FR GB IT LI LU NL SE**

(71) Anmelder: **BAYER AG, Zentralbereich Patente, Marken und Lizenzen, D-5090 Leverkusen 1, Bayerwerk (DE)**

(72) Erfinder: **Schmidt, Gunter, Dr., Pahlkestrasse 63, D-5600 Wuppertal 1 (DE)**
Erfinder: **Zeiler, Hans-Joachim, Dr., Elsbeekerstrasse 46, D-5620 Velbert 15 (DE)**
Erfinder: **Metzger, Karl Georg, Dr., Pahlkestrasse 15, D-5600 Wuppertal 1 (DE)**

## (54) 7-Alpha-Aminoacyl-3-chlorcephalosporine, Verfahren zu ihrer Herstellung sowie ihrer Verwendung als Arzneimittel.

(57) Die Erfindung betrifft ein neues Cepahlosporin der Formel

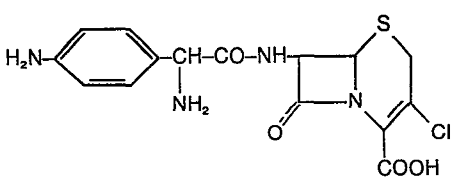

und dessen pharmazeutisch verträgliche, nicht toxische Salze, ein Verfahren zu seiner Herstellung durch Umsetzung von α-Amino-α-4-aminophenylessigsäure mit

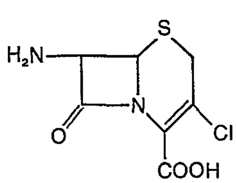

sowie seine Verwendung bei der Bekämpfung von Infektionskrankheiten.

EP 0 090 240 A2

0090240

BAYER AKTIENGESELLSCHAFT 5090 Leverkusen, Bayerwerk

Zentralbereich
Patente, Marken und Lizenzen Dn/Kü-c

# 7-α-Aminoacyl-3-chlorcephalosporine, Verfahren zu ihrer Herstellung sowie ihrer Verwendung als Arzneimittel

Die vorliegende Erfindung betrifft neue 7-α-Aminoacyl-3-chlorcephalosporine, ein Verfahren zu ihrer Herstellung sowie ihre Verwendung als Arzneimittel, insbesondere als oral wirksame Antibiotika.

Verschiedene 7-α-Aminoacylcephalosporin-Antibiotika mit unterschiedlichen Substituenten in der 3-Stellung des Moleküls sind bekannt. Zum Beispiel die ß-Lactam-Antibiotika Cephalexin, 7-(D-α-Phenylglycylamido)-3-methyl-3-cephem-4-carbonsäure (GB-PS 1 174 335) und Cefaclor, 7-(D-α-Phenylglycylamido)-3-chlor-3-cephem-4-carbonsäure (DE-OS 2 408 698 und DE-OS 2 728 578). Weiterhin sind eine Reihe von α-Aminoacylcephalosporinverbindungen beschrieben worden, deren Moleküle in der 3-Stellung entweder eine Acetoxymethylgruppe oder eine heterocyclische Thiolgruppe tragen, z.B. Cefatrizin (DE-OS 2 364 192). In der CH-PS 625 247 sind 7-ß-/D-2-Amino-2-(aminophenyl) acetylamino/-cephemcarbonsäuren und ihre Alkylsulfonylderivate beschrieben.

Die vorliegende Erfindung stellt das Cephalosporin der folgenden Strukturformel

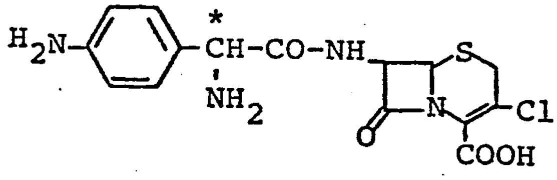

und dessen nicht toxische, pharmazeutisch verträglichen Salze zur Verfügung.

Wegen der Anwesenheit des mit * bezeichneten asymmetrischen Kohlenstoffatoms schließt das 3-Chlorcephalosporin der obigen Formel die D-, L- und DL-Form ein. Die D-Form ist die erfindungsgemäß bevorzugte isomere Form.

Das neue Cefalosporin kann nach der gemischten Anhydrid-Methode aus dem (Benzyloxycarbonyl)- bzw. tert.-Butyloxycarbonyl-geschützten p-Aminophenylglycin und der 7-Amino-3-chlor-3-cephem-4-carbonsäure (7-ACCS) mit anschließender hydrogenolytischer bzw. acidolytischer N-Demaskierung erhalten werden.

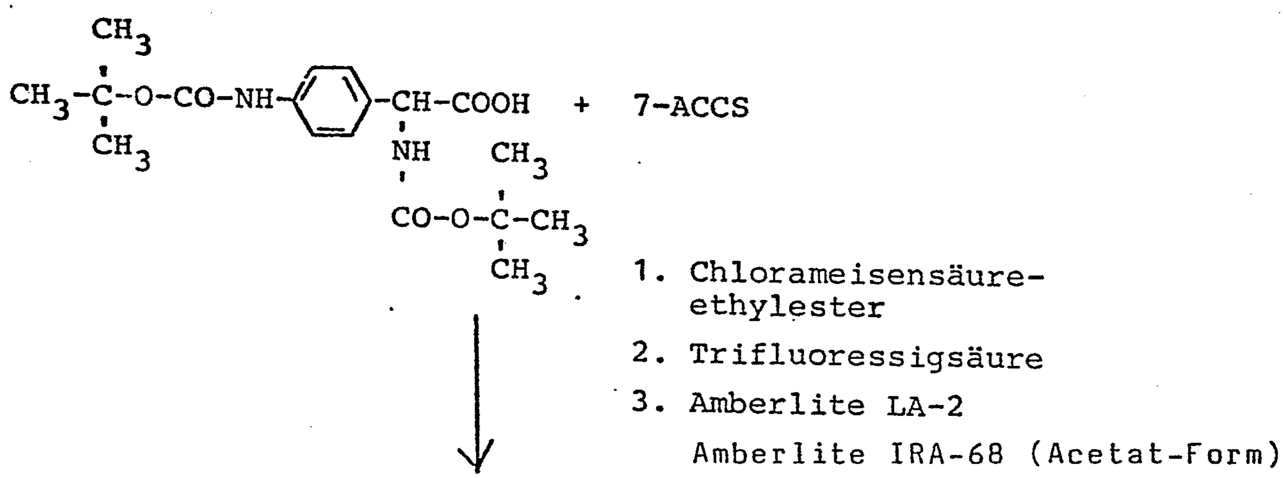

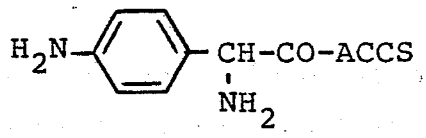

Die Verbindung kann in zwitterionischer Form vorliegen, die sich durch eine intramolekulare Salzbildung ergibt. Der Ausdruck "pharmazeutisch verträgliche, nichttoxische Salze" umfaßt sowohl die Salze der $C_4$-Carbonsäuregruppe als auch die Säureadditionssalze der Aminogruppen der Seitenkette, vorzugsweise der $\alpha$-Aminogruppe.

Pharmazeutisch verträgliche Salze der $C_4$-Carbonsäuregruppe schließen die mit anorganischen Basen gebildeten Salze ein, wie die Natrium-, Kalium- und Calciumsalze, die mit Natriumbicarbonat, Kaliumcarbonat, Calciumhydroxid oder Natriumhydroxid hergestellt werden können.

Pharmazeutisch verträgliche Aminsalze können z.B. mit organischen Aminen, wie Dicyclohexylamin,Benzylamin, 2-Aminoethanol, Diethanolamin oder Diisopropylamin hergestellt werden. Die Säureadditionssalze der Amino-Gruppen umfassen die mit Mineralsäure gebildeten Salze, wie die Hydrochloride, die Hydrobromide und die Sulfate, sowie die mit organischen Sulfonsäuren gebildeten, wie die p-Toluolsulfonate.

Im allgemeinen kann man für die Acylierung der 7-Amino-3-cephem-4-carbonsäure irgendwelche der bekannten Peptidverknüpfungsmethoden anwenden. Wenn z.B. als aktiviertes

Derivat ein Säurehalogenid eingesetzt wird, erfolgt die Acylierung in Gegenwart eines Halogenwasserstoffacceptors, wie z.B. Natriumbicarbonat, Pyridin.

Wenn man die N,N'-geschützen p-Aminophenylglycine einsetzt, kann die Acylierung in Gegenwart eines Kondensationsmittels, wie z.B. N,N'-Dicyclohexylcarbodiimid oder N-Ethyl-N'-(3-dimethylaminopropyl)-carbodiimid erfolgen.

Neben der Benzyloxycarbonyl- und tert.-Butyloxycarbonylgruppe können auch andere Aminoschutzgruppen, beispielsweise die p-Nitrobenzyloxycarbonyl-, Trichlorethoxycarbonyl-, Trityl- oder 2-(p-Biphenyl)-isopropyloxycarbonyl gruppe oder ß-Dicarbonylverbindungen (z.B. der 1-Methyl-2-ethoxycarbonyl-vinyl-, oder 1-Methyl-2-benzoyl-vinyl-Rest) Anwendung finden.

Die Acylierung erfolgt in einem inerten Lösungsmittel, z.B. in Tetrahydrofuran, Dimethylformamid, Aceton, Acetonitril oder Methylenchlorid und vorzugsweise bei einer Temperatur zwischen -40 und +20°C.

Die DL-α-Amino-α-(p-aminophenyl)-essigsäure kann z.B. aus p-Nitrotoluol nach folgendem Syntheseschema erhalten werden:

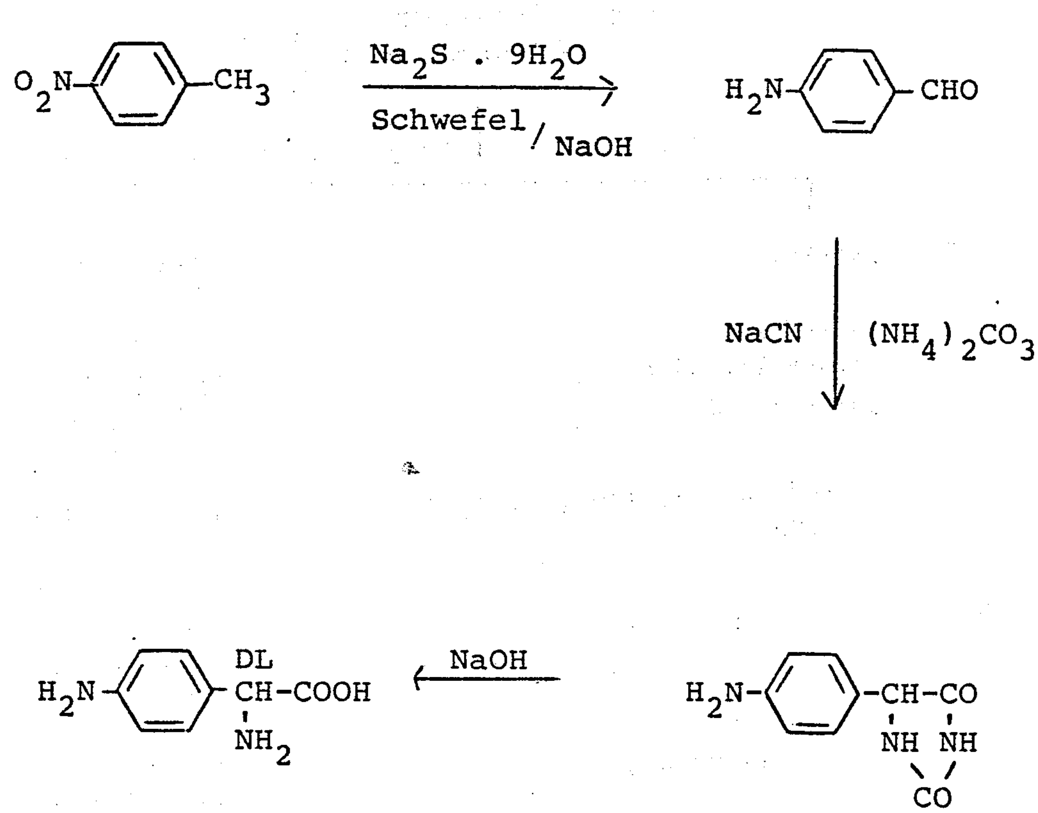


Aus der DL-Form können die reinen D- und L-Formen nach folgendem Formelschema erhalten werden.

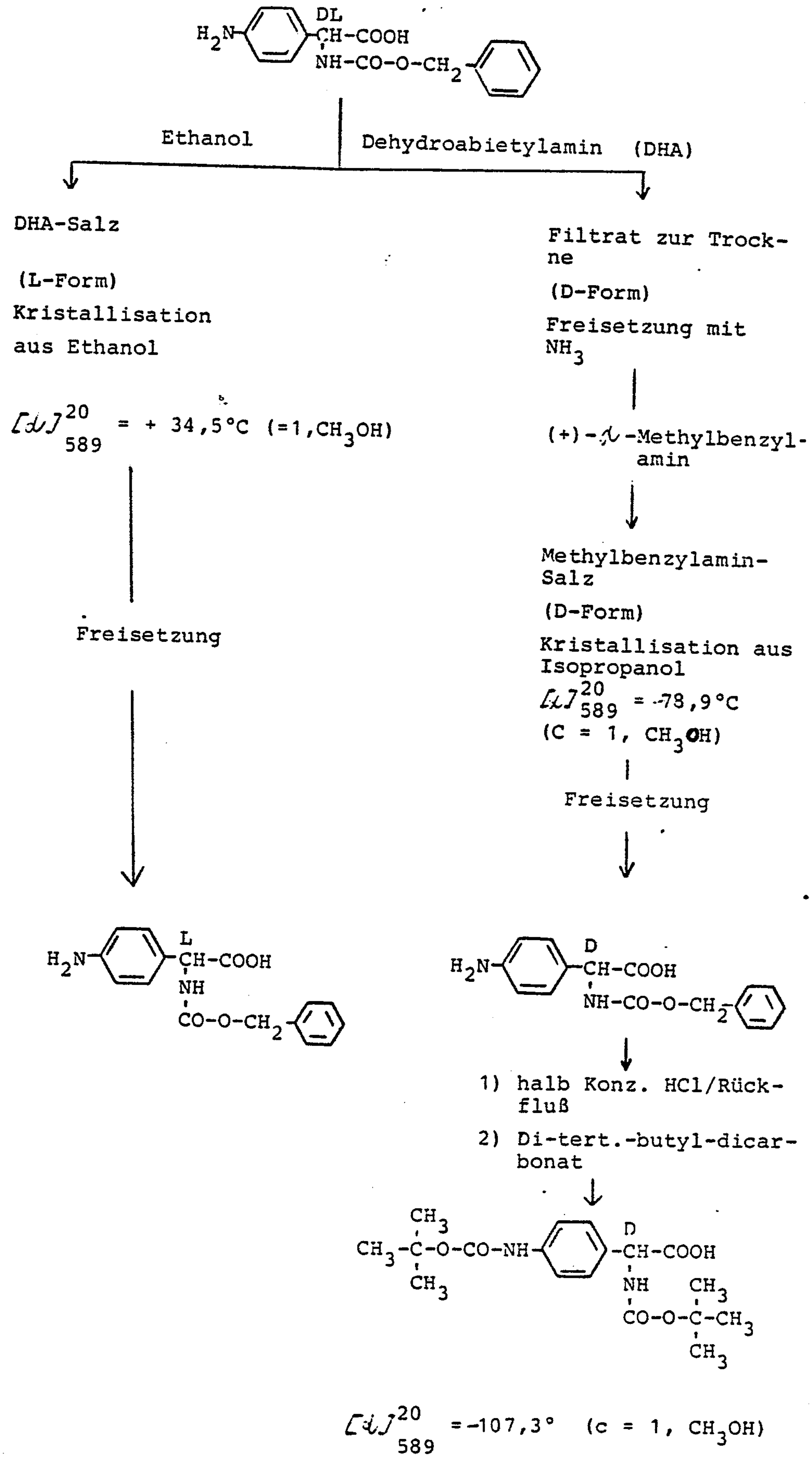
Ethanol
Dehydroabietylamin (DHA)
DHA-Salz
(L-Form)
Kristallisation
aus Ethanol
[α]$^{20}_{589}$ = + 34,5°C (=1,$CH_3OH$)
Freisetzung
Filtrat zur Trockne
(D-Form)
Freisetzung mit $NH_3$
(+)-α-Methylbenzylamin
Methylbenzylamin-Salz
(D-Form)
Kristallisation aus Isopropanol
[α]$^{20}_{589}$ = -78,9°C
(C = 1, $CH_3OH$)
Freisetzung
1) halb Konz. HCl/Rückfluß
2) Di-tert.-butyl-dicarbonat
[α]$^{20}_{589}$ =-107,3° (c = 1, $CH_3OH$)

Die 7-Amino-3-chlor-3-cephem-4-carbonsäure kann man entweder nach J. Elks, Recent Advances in the Chemistry of ß-Lactam-Antibiotics", 28, 243 - 251 (1977) oder aus dem Handelsprodukt Cefaclor durch enzymatische Abspaltung von D-Phenylglycin erhalten.

Das erfindungsgemäße Cephalosporin besitzt ein breites antibakterialles in vitro-Wirkungsspektrum. Bei Serratia- Providencia- und Proteusstämmen wird eine verbesserte Wirkung im Vergleich zu Cefaclor gefunden.

So sind die neuen Verbindungen in vitro gegen

| | |
|---|---|
| E. coli | T 7 |
| | A 261 |
| | Neum. |
| | 183/58 |
| | F 41 |
| | C 165 |
| | 4 322 |

und gegen

| | |
|---|---|
| Klebs. | 57 US |
| | 63 |
| | 1 852 |
| | 6 097 |

in einem Dosisbereich von 0,5 - 8 µg/ml wirksam.

Bei parenteraler oder insbesondere oraler Verabreichung sind die neuen p-Amino-cefaclorderivate gegen Mikroorganismen, wie Staphylococcus aureus 133, Streptococcus fall. 27 lol, Enterococcus AT CC 9790 und gram-negative Bakterien, z.B. Escherichia coli und Klebsiella sehr gut wirksam.

Beispiel 1

1.1 5 - (4-Aminophenyl)-2,4-imidazolidin-dion

90 g (Trockensubstanz ≙ 0,495 mol) wasserfeuchter p-Aminobenzaldehyd, gelöst in 1000 ml Methanol, wurden zu einer Lösung von 37,0 g (0,75 mol) Natriumcyanid und 192,9 g (2,02 mol) Ammoniumcarbonat in 1000 ml Wasser zugetropft und 20 Stunden bei 60°C gerührt, wobei eine klare Lösung entstand. Nach Abdestillieren des Methanols im Vakuum wurde die Restlösung bei 0°C mit konzentrierter Salzsäure auf pH 2 angesäuert, 10 Minuten gerührt und anschließend mit 4 N NaOH auf pH 7 eingestellt. Das ausgefallene Produkt wurde abgesaugt mit Wasser gewaschen und bei 60°C im Vakuum getrocknet.

Ausbeute: 69,4 g (73 %)

$C_9H_9N_3O_2$ (191,2)

| | | | | | | |
|---|---|---|---|---|---|---|
| Ber. | C | 56,54 | H | 4,74 | N | 21,99 |
| Gef. | C | 56,8 | H | 4,7 | N | 21,8 |

$^1$H-NMR (/$D_6$/ DMSO, 60 MHz): $\delta$ = 4,95 (S; 5-H, Imidazolidin), 6,65 (d; J = 8,3 Hz, 3-H, 5-H), 7,03 ppm (d; J = 8,3 Hz, 2-H, 6-H).

1.2 DL-$\alpha$-Amino-$\alpha$-(4-aminophenyl)essigsäure

70 g (0,336 mol) 5-(4-Aminophenyl)-2,4-imidazoli-

dindion wurden in 700 ml 10 %iger Natronlauge unter Rühren 30 Stunden auf 100°C erhitzt. Man kühlte auf 0°C ab, säuerte mit konzentrierter Salzsäure auf pH 2 an und rührte noch 15 Minuten nach. Anschließend wurde die Lösung mit 4 N NaOH bei Eiskühlung auf einen pH-Wert von 5,0 eingestellt. Das hellgelbe ausgefallene Material wurde abgesaugt, mit Eiswasser und danach mit Aceton gewaschen und bei 60°C getrocknet.

Ausbeute: 57,3 g (94 %)

$C_8H_{10}N_2O_3$ (166,2)

| | | | | | | |
|---|---|---|---|---|---|---|
| Ber. | C | 57,82 | H | 6,06 | N | 16,86 |
| Gef. | C | 57,4 | H | 6,4 | N | 16,6 |

$^1$H-NMR ($[D_6]$ DMSO/NaOD, 60 MHz): $\delta$ = 4,1 (s; α-CH), 6,63 (d; J = 8,3 Hz, 3-H, 5-H), 7,15 ppm (d; J = 8,3 Hz, 2-H, 6-H).

1.3 DL-α-tert.-Butyloxycarbonylamino-α-(4-tert.-butyloxycarbonylaminophenyl)essigsäure

25 g (0,1 mol) D-α-Amino-α-(4-amino-phenyl)essigsäure dihydrat (Gehalt; 80,7 %) wurden in 150 ml Wasser suspendiert, mit 150 ml 2 N NaOH in Lösung gebracht und mit 300 ml Dioxan versetzt. Anschließend wurden 64,5 g (0,3 mol) Di-tert.-butyldicarbo-nat in 30 Minuten zugetropft und über Nacht gerührt. Anderntags wurde Dioxan abdestilliert. Die Restlösung wurde mit $H_2O$ verdünnt, mit Essigester gewaschen und mit 2 N HCl bei Eiskühlung auf pH 2 in Gegenwart von Essigester angesäuert. Nach Waschen mit 10 prozentiger Kochsalzlösung, Trocknen über $Na_2SO_4$ und Einengen der organischen Phase wurde der Rückstand aus heißen Essigester unter Zugabe von Petrolether kristallisert.

Ausbeute: 30,4 g (83 %)

$C_{18}H_{26}N_2O_6$ (366,4)

| | | | |
|---|---|---|---|
| Ber. | C 59,01 | H 7,15 | N 7,64 |
| Gef. | C 58,9 | H 7,3 | N 6,8 |

$^1$H-NMR ([$D_6$] DMSO, 200 MHz): $\delta$ = 1,43 (d; 18 H), 4,98 (d; $\alpha$-CH) 7,27 - 7,4 ppm (AB-System; 4 H, Phenyl).

1.4 7-[DL - $\alpha$-tert.-Butyloxycarbonylamino-$\alpha$-(4-tert.-butyloxycarbonylaminophenyl)-acetamido]-3-chlor-3-cephem-4-carbonsäure

4,0 g (10,9 mmol) DL -$\alpha$-tert. Butyloxycarbonylamino-$\alpha$-(4-tert.-butyloxycarbonylaminophenyl)essigsäure wurden in 50 ml THF mit 1,53 ml (10,9 mmol) Triethylamin, 3 Tropfen N-Methylmorpholin versetzt und bei -30°C 1,43 ml (10,9 mmol) Chlorameisen-säure isobutylester zugegeben. Nach 60 Minuten bei -30° bis -10°C gab man eine vorgekühlte Triethylamin Salzlösung von 2,8 g (12 mmol) 7-Amino-3-chlor-3-cephem-4-carbonsäure in THF/Wasser (3:1) gelöst, hinzu. Man ließ 2 Stunden ohne Kühlung nachrrühren und stellte den pH-Wert bei 7,2 ein. Man versetzte mit Essigester/Wasser und säuerte bei 0°C auf pH 2 an. Die Essigesterphase wurde abgetrennt, gewaschen, getrocknet, bis auf 80 ml eingeengt und in Petrolether eingerührt. Das ausgefallene Produkt wurde abgesaugt und getrocknet.

Ausbeute: 3,3 g (52 %)

$C_{25}H_{31}ClN_4O_8S$ (583,1)

IR-Spektrum: 1775 (s), 1700 (s), 1500 (s), 1358 (m), 1230 (s), 1150 (s), 1040 $cm^{-1}$ (s) (Chloroform).

$^1H$ - NMR (/$\bar{D}_6$/ DMSO, 250 MHz): $\delta$ = 1,4 - 1,48 (d; 18 H), 3,62 - 4,1 (mm; 2H, 2-H), 5,12 - 5,26 (breites m; $\alpha$-CH, 6-H), 5,56 - 5,82 (mm; 7-H), 7,3 - 7,46 (mm; 4H, Phenyl), 9,18 - 9,28 (q; -NH-), 9,41 ppm (s; -NH-).

1.5 7-ß-/$\bar{D}$L-(4-Aminophenyl)-glycinamido/-3-chlor-$\Delta^3$-cephem-4-carbonsäure

3,3 g (5,7 mmol) Di-tert.-butyloxycarbonyl-geschütztes Cephalosporinderivat wurden mit 40 ml Trifluoressigsäure (TFE) und 0,4 ml Anisol 15 Minuten bei 0° - 10°C behandelt. Anschließend wurde TFE im Vakuum abdestilliert und der ölige Rückstand mit Ether versetzt.

Die kristalline Verbindung wurde abgesaugt, mit Ether gewaschen und getrocknet. Das Trifluoracetat-Salz wurde in 200 ml Wasser und 200 ml Ether gelöst und mit Amberlite LA-2 (J.L. Spencer, E.H. Flynn, R.W. Roeske, F.Y. Siu und R.R. Chauvette, J. Med. Chem. 9, 746, (1966)) in Ether tropfenweise bis pH 5,2 unter Rühren versetzt.

Die Etherphase wurde abgetrennt, die wäßrige Phase mehrmals mit Ether gewaschen und gefriergetrocknet.

Ausbeute: 1,6 g (35 %)

$C_{15}H_{15}ClN_4O_4S \cdot 2H_2O$ (418,8)

| | | | | | |
|---|---|---|---|---|---|
| Ber. | C 43,2 | H 4,57 | N 13,38 | S 7,67 | Cl 8,46 |
| Gef. | C 44,0 | H 4,7 | N 12,9 | S 6,8 | Cl 7,5 |

IR-Spektrum: 1775 (s), 1680 (w), 1605 (s), 1460 (s), 1360 $cm^{-1}$ (s) Nujol.

$^1$H-NMR (DCOOD, 200 MHz): δ = 3,48 - 3,98 (mm; 2H, 2-H), 5,22 - 5,32 (dd; 6-H), 5,65 (d; α-CH), 5,8 - 5,9 (dd; 7-H), 7,72 - 7,87 ppm (m; 4H, Phenyl).

1.6 7-Amino-3-chlor-3-cephem-4-carbonsäure aus Cefaclor mit Penicillinacylase-Harz

35,4 g Cefaclor wurden 60 Minuten in 1000 ml entsalztem Wasser, das mit Triethylamin auf pH 7,5 eingestellt ist, unter Rühren suspendiert. Die Suspension wurde über ein AS-Filter (von Seitz) abgesaugt. Der pH-Wert der Lösung wurde mit 18 %iger HCl auf 6,8 eingestellt. Durch Zugabe von 120 g Penicillinacylase IR C - 50 - Harz (12,2 Units/g) wurde D-Phenylglycin bei 25$^{o}$C unter Neutralisation mit Triethylamin bei einem konstanten pH-Wert von 6,8 in 5 Stunden abgespalten. Das Penicillinacylase-Harz wurde abfiltriert, mit Wasser gewaschen und die Spaltlösung im Vakuum auf ca. 100 ml konzentriert. Das ausgefallene D-Phenylglycin wurde abfiltriert, mit Aceton gewaschen und getrocknet. Die Lösung wurde mit 18 %iger HCl auf pH ca. 3,7 eingestellt, der Niederschlag abgesaugt, mit entionisiertem Wasser, Aceton gewaschen und getrocknet.

Ausbeute: 14,9 g (79 %)

$C_7H_7Cl\ N_2O_3S$ (234,6)

| | | | | | |
|---|---|---|---|---|---|
| Ber. | C 35,82 | H 3,01 | N 11,93 | S 13,66 | Cl 15,11 |
| Gef. | C 36,0 | H 3,1 | N 11,5 | S 12,85 | Cl 14,3 |

$^1$H-NMR ($NaDCO_3$, 250 MHz); $\delta$ = 3,54 (d; 2-H), 3,92 (d; 2-H), 5,14 (d; 6-H), 5,46 ppm (d; 7-H).

Beispiel 2

2.1 DL-$\alpha$-Benzyloxycarbonylamino-$\alpha$-(4-aminophenyl) essigsäure

30 g (0,142 mol) 88 proz. DL-$\alpha$-Amino-$\alpha$-(4-aminophenyl)essigsäure wurden in 250 ml Wasser suspendiert und mit 11,9 g (0,142 mol) $NaHCO_3$ auf 100°C erwärmt. Anschließend wurde noch 2 N NaOH bis pH 9 zugegeben (fast klare Lösung). 40,6 g (0,143 mol) Benzyl-p-nitrophenyl-carbonat (Y. Wolman, D. Ladkany und M. Frankel. J. Chem. Soc. 1967, 689), gelöst in 150 ml Dioxan, wurden zugegeben und 2 Stunden bei 100°C gerührt. Die Lösung wurde heiß filtriert, das Filtrat im Vakuum von Dioxan befreit und die Lösung zweimal mit Essigester gewaschen.

Die wässrige Phase wurde im Vakuum vom Essigester befreit und bei 10° mit 2N HCl auf pH 4,5 angesäuert. Das ausgefallene Produkt wurde abgesaugt, mit Ethanol 1 Stunde heiß digeriert, abgesaugt und mit Ether gewaschen.

Ausbeute: 27,5 g (63 %)

$C_{16}H_{16}N_2O_4$ (309,3)

| | | | |
|---|---|---|---|
| Ber. | C 63,99 | H 5,37 | N 9,33 |
| Gef. | C 63,2 | H 5,4 | N 9,2 |

$^1$H-NMR ($[D_6]$ DMSO, 250 MHz): δ = 5,02 (d; α-CH), 5,09 (s; Benzyl-$CH_2$), 6,6 (d; 3-H, 5-H), 7,13 (d; 2-H, 6-H), 7,33 - 7,42 (m; 5H, Phenyl), 7,92 ppm (d; -NH-).

2.2 Dehydroabietylaminsalz der L - α-Benzyloxycarbonylamino-α-(4-aminophenyl)-essigsäure

20 g (66,8 mmol) DL-α-Benzyloxycarbonylamino-α-(4-aminophenyl)essigsäure wurden in 300 ml Ethanol suspendiert und mit 21,1 g (66,2 mmol) Dehydroabietylamin (90 proz), gelöst in 300 ml Ethanol, versetzt. Nach 15 Minuten Kochen entstand eine klare Lösung, die man langsam auf Raumtemperatur abkühlen ließ. Der gebildete Niederschlag wurde abgesaugt und mit Ethanol gewaschen. (Filtrat für (+)-α-Methylbenzyl-aminsalzbildung).

Ausbeute: 20 g

Nach dreimaligem Umkristallisieren aus Ethanol erhielt man 7,5 g (38,5 %)

$[\alpha]^{20°}_{589}$ = + 34,5° (c = 1, $CH_3OH$)

$C_{36}H_{47}N_3O_4$ (585,8)

Ber. C 73,81 H 8,08 N 7,17
Gef. C 73,7 H 8,2 N 7,2

2.3 (+)-α-Methylbenzylaminsalz der D-α-Benzyloxycarbonylamino-α-(4-aminophenyl)essigsäure

Das ethanolische Filtrat des Dehydroabiethylaminsalzes wurde bis zur Trocknung eingeengt, der Rückstand im Wasser aufgenommen (150 ml) und mit 50 ml konzentriertem Ammoniak versetzt.

Die milchige Suspension wurde zweimal mit Ether extrahiert und die klare wäßrige Phase bis zur Trocknung eingeengt und bei 40°C getrocknet. 10,5 g (33,2 mmol) des rohen Ammoniumsalzes der Benzyloxycarbonyl-geschützten p-Aminophenylglycinverbindung wurden in 50 ml Wasser und 100 ml Ethanol gelöst, mit 4,0 g (33,2 mmol) D-(+)-α-Methylbenzylamin versetzt und 15 Minuten in der Hitze gerührt, bis eine klare Lösung entstanden war. Die Lösung wurde filtriert, im Vakuum bis zur Trockne eingeengt und 3 Stunden bei 40°C getrocknet. Der Rückstand wurde dreimal aus Isopropanol umkristallisiert.

Ausbeute: 5,1 g (37 %)

$[\alpha]^{20°}_{589}$ = - 78,9° (c = 1, $CH_3OH$).

$C_{24}H_{27}N_3O_4$ (421,5)

Ber. C 69,39 H 6,45 N 9,97
Gef. C 68,2 H 6,4 N 9,8

2.4 7 ß-/D-(4-Aminophenyl)-glycinamido7-3-chlor-$\Delta^3$ - cephem-4-carbonsäure

Das D-(+)-α-Methylbenzylaminsalz wurde in Wasser suspendiert, mit konzentrierter $NH_3$-Lösung auf pH 10,5 eingestellt, mit Ether extrahiert und die wäßrige Phase bis zur Trocknung eingeengt. Der Trockenrückstand wurde mit halbkonzentrierter Salzsäure 30 Minuten unter Rückfluß erhitzt, die wäßrige Lösung einmal mit Ether extrahiert und bis zur Trocknung eingeengt. Man erhielt das Hydrochlorid der D-α-Amino-α-(4-aminophenyl)essigsäure. Wie im Beispiel 1.3 beschrieben wurde mit Hilfe von Di-tert.-butyldicarbonat das Di-tert.-Butyloxycarbonyl geschützte p-Aminophenylglycin hergestellt. 7,0 g (19,1 mmol) D-α-tert.-Butyloxycarbonylamino -α-(4-tert.butyloxycarbonylaminophenyl)-essigsäure wurden in 75 ml THF mit 2,67 ml (19,1 mmol) Triethylamin 3 Tropfen N-Methylmorpholin versetzt und bei -30°C 1,84 ml (19,1 mmol) Chlorameisensäureethylester zugegeben.

Nach 60 Minuten Rühren bei -30° bis -10°C gab man eine vorgekühlte Triethylamin-Salzlösung von 4,93 g

(21 mmol) 7-Amino-3-chlor-3-cephem-4-carbonsäure hinzu, die durch Verrühren in 70 ml THF und 15 ml Wasser unter Zugeben einer 10 %igen Triethylaminlösung in THF hergestellt wurde.

Man ließ 2 Stunden ohne Kühlung nachrühren und stellte den pH-Wert bei 7,2 ein. Man versetzte mit Essigester/Wasser und säuerte bei 0°C auf pH 2 an. Die Essigesterphase wurde abgetrennt, gewaschen, getrocknet, bis auf 100 ml eingeengt und in Petrolether eingerührt. Das ausgefallene Produkt (5,5 g) wurde abgesaugt, mit 60 ml Trifluoressigsäure und 0,8 ml Anisol 15 Minuten bei 0°C bis 10°C behandelt.

Das Trifluoracetat-Salz, erhalten durch Etherfällung, wurde in 300 ml Wasser/300 ml Ether mit Amberlite LA-2 bis pH 5,2 verrührt. Die wäßrige Lösung wurde mehrmals mit Ether gewaschen und gefriergetrocknet.

Ausbeute: 4,9 g (61 %)

IR-Spektrum: 1780 (s), 1695 (m), 1620 (m), 1470 (s), 1385 (s), 1190 (w) Nujol.

$^1$H-NMR (DCOOD, 250 MHz): $\delta$ = 3,57 - 3,98, (d d ; 2 H, 2-H), 5,33 (d; 6-H), 5,73 (s; $\alpha$-CH), 5,96 (d; 7-H), 7,84 - 7,94 ppm ( q; 4H, Phenyl).

## Patentansprüche

1) Verbindung der Formel

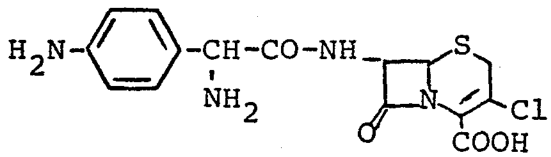

und deren pharmazeutisch verträgliche, nicht toxische Salze.

2) Verbindung nach Anspruch 1 in der D-Form und ihre pharmazeutisch verträglichen nicht toxischen Salze.

3) Verfahren zur Herstellung der Verbindung der Formel

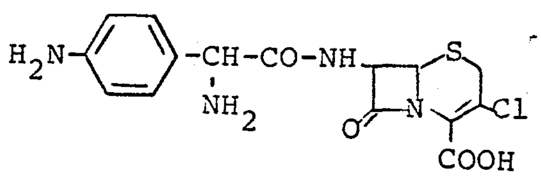

dadurch gekennzeichnet, daß man α-Amino-α-(4-amino-phenyl)essigsäure mit

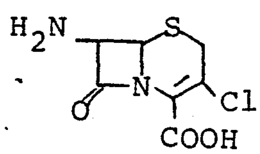

umsetzt.

4) Verwendung der Verbindung nach Anspruch 1 und ihrer pharmazeutisch verträglichen nicht toxischen Salze bei der Bekämpfung von Infektionskrankheiten.